# EUROPEAN PATENT APPLICATION

(11) **EP 4 197 453 A1**
(43) Date of publication of application: **21.06.2023**
(21) Application number: 21896082.1
(22) Date of filing: 25.02.2021
(51) Int. Cl.: A61B 17/04, A61F 2/08

(54) **LOOP AND MANUFACTURING METHOD FOR SAME**

(30) Priority: 30.11.2020 CN 202011374002
(71) Applicant: Shanghai Orthopair Medical Co., Ltd., Shanghai 201201 (CN)
(72) Inventor: WANG, Yuanqiang, Shanghai 201201 (CN); YUAN, Zheng, Shanghai 201201 (CN); NIE, Jiali, Shanghai 201201 (CN)
(74) Representative: Bayramoglu et al.
(86) International application number: PCT/CN2021/077884
(87) International publication number: WO 2022/110562

(57) **Abstract**

Provided by the present invention is a loop, comprising a fixing plate and a suture, wherein the fixing plate is provided with at least two threading holes, the suture is wound to form a first coil and a second coil, the first coil and the second coil are arranged on a same side of the fixing plate, and two ends of each of the first coil and the second coil are configured to pass through the threading holes; the two ends of the first coil are movably connected to form a first adjustable portion, a third end of the second coil is continuously connected to a first end of the first coil to form a fixing portion, the fixing portion is configured to compress a fourth end of the second coil to form a second adjustable portion. By providing the first adjustable portion and the elastic portion, not only the purpose of automatically adjusting the length of the coil in the loop is achieved, but also the coil in the loop cannot be enlarged and retreated due to the pulling force of a graft when no artificial external force exists, the operation time is shortened, and the operation risk is reduced. A manufacturing method of the loop is further provided by the present invention.

## Description

### FIELD OF TECHNOLOGY

The present invention relates to the field of medical devices, and more specifically, to a loop and a manufacturing method of the loop.

### BACKGROUND

Loops are used for fixing ligaments or tendons and bones in orthopedic reconstruction, and are commonly used for anterior and posterior cruciate ligament reconstruction of the knee joints, acromioclavicular joint dislocation fixation and like. For example, in cruciate ligament surgery, bone tunnels need to be drilled in femur and tibia respectively, and a ligament graft without bone blocks at both ends is secured in the bone tunnels of the femur and tibia using a titanium plate with a loop and screws.

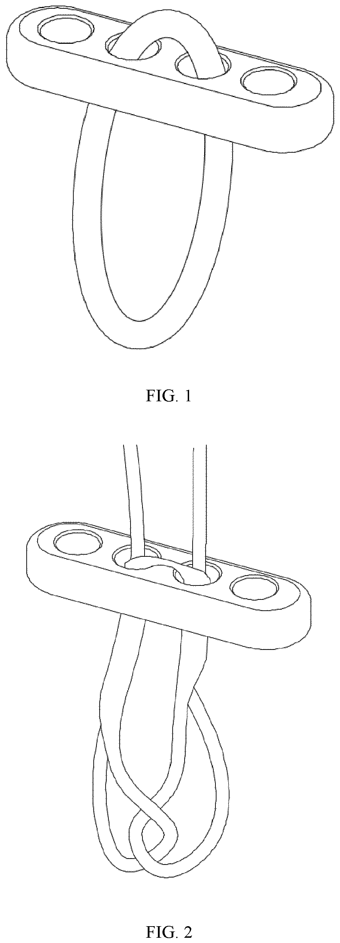

In the cruciate ligament reconstruction of the knee joints, the ligament graft should fill the femoral tunnel as completely as possible, and existing titanium plates with the loop are divided into a length-fixed titanium plate with a loop and an adjustable titanium plate with a loop. FIG. 1 is a structure diagram of a fixed-length titanium plate with a loop in the prior art. Referring to FIG. 1, a coil of the fixed-length titanium plate with the loop is usually of fixed specifications in different sizes, has no ability of adjusting the length of the coil, needs measurement and calculation before use, and is hard to completely fill the femoral tunnel, which may increase unnecessary surgery time and has surgical risks, as well as requires more space in the operating room for storage. FIG. 2 is a structure diagram of an adjustable titanium plate with a loop in the prior art. Referring to FIG. 2, the adjustable titanium plate with the loop can solve the problem of the fixed-length titanium plate with the loop. However, the product currently available on the market is not conducive to surgery due to the fact that the reaction force of tendon and other grafts makes the coil easily retreat; and the processing technology is complicated, and requires special equipment for winding and weaving to meet the same mechanical strength as the fixed-length titanium plate with the loop, resulting in high investment cost.

The patent application with publication number of CN109350149A discloses a cruciate ligament repairing structure and method. The cruciate ligament repairing structure comprises a fixing plate and a suture. The fixing plate is provided with two line holes; the number of the sutures is two, the sutures penetrate through one of the line holes, then penetrate out of the other of the line holes after being wound to form a coil, and finally penetrate back to the first penetrated line hole; the line holes through which the two sutures penetrate are different, and the ends of the two sutures which are wound to form the coil are connected. In the cruciate ligament repairing structure provided by this present invention, the length of the coil can be adjustable after winding the two sutures to form the coil, the situation that the fixation effect of the titanium plate with a loop is affected due to the problems of drilling and the length of a graft in the surgery is avoided, and the cruciate ligament repairing structure has the advantages of being low in cost and high in safety. However, the cruciate ligament repairing structure is not provided with an anti-retreating structure, the coil is easy to retreat under the reaction force of the graft, which is not conducive to the surgery.

Therefore, it is necessary to design a novel loop to avoid the problems in the prior art.

### SUMMARY

An objective of the present invention is to provide a loop and a manufacturing method of the loop to solve the problems that in the prior art, the length of a coil in a fixed-length type titanium plate with a loop cannot be adjusted while a coil in an adjustable type titanium plate with a loop is easy to retreat under the reaction force of a graft, leading to inconvenience in the surgery.

To achieve the objective, in a first aspect, a loop is disclosed, comprising a fixing plate and a suture, wherein the fixing plate is provided with at least two threading holes, the suture is wound to form a first coil and a second coil, the first coil and the second coil are arranged on the same side of the fixing plate, and two ends of each of the first coil and the second coil both are configured to pass through the threading holes; two ends of the first coil are respectively defined as a first end and a second end, and the first end of the first coil and the second end of the first coil are movably connected therewith to form a first adjustable portion; two ends of the second coil are respectively defined as a third end and a fourth end, the third end of the second coil is connected to the first end of the first coil to form a fixing portion, and the fixing portion is configured to compress the fourth end of the second coil to form a second adjustable portion.

The loop of the present invention has the following beneficial effects: (1) the third end of the second coil is connected to the first end of the first coil to form the fixing portion, and the fixing portion is configured to compress the fourth end of the second coil to form the second adjustable portion. On the one hand, the second adjustable portion can achieve self-locking under the action of pulling force of a graft, which not only can effectively prevent the second coil from being retreated, but also can achieve self-locking without the operation of external force, and the duration of surgery is saved. And on the other hand, it is guaranteed that the first coil cannot be enlarged and retreated by the action of pulling force of the graft without the action of artificial external force, so that the entire loop cannot be retreated under the reaction force of the graft, the smooth surgery is guaranteed and the surgical risk is reduced; (2) the length of the coil in the loop can be automatically adjustable by the first adjustable portion under the action of the graft to guarantee that the coil length of the fist coil is the same as the coil length of the second coil, so that no redundant calculation is required during surgery, the surgery time is shortened, and the surgical risk is reduced; (3) the loop comprises the first coil and the second coil, and the first coil and the second coil are arranged on the same side of the fixing plate to share the pulling force of the graft, so that the overall stress strength of the loop is increased, the loop becomes firmer, and this can meet the clinical requirements on the structural mechanics and be suitable for fixing the grafts with different pulling force; and (4) the structure is simple, the design is ingenious, and the production cost input is low.

Preferably, the first coil and the second coil are woven by one continuous suture configured to pass through the threading holes of the fixing plate. The beneficial effects include: the first coil, the second coil and the fixing portion are of a continuous suture, the loop becomes firmer and more durable, thus preventing the joint of the coil from being broken to affect the use.

Preferably, the second end of the first coil is provided with a nesting portion, the first end of the first coil is movably connected to the second end of the first coil by passing through the nesting portion to form the first adjustable portion. The beneficial effects include: the first end of the first coil can pass through the nesting portion simply and easily, thus facilitating the manufacturing of the loop.

Preferably, the first end of the first coil and the second end of the first coil are movably connected by a threading process to form the first adjustable portion. The beneficial effects include: due to the own weaving process of the suture, the outer surface roughness of the suture is high, and the line hole in the suture line is of a rough inner surface; the outer surface of the suture at the first end of the first coil is in interference fit with the line hole in the suture of the second end of the first coil by means of the threading process, thus providing an anti-retreating effect by the friction force therebetween.

Preferably, the first end of the first coil is movably connected to the second end of the first coil by a fastening, a clamping or binding method to form the first adjustable portion. The beneficial effects include: the movable connection by the fastening, the clamping or binding method is simple and easy, thus facilitating the manufacturing of the loop.

Preferably, the two ends of the first coil are configured to pass through a common threading hole, and the threading hole through which the two ends of the first coil pass are configured to is different from the threading hole through which the third end of the second coil is configured to pass. The beneficial effects include: the two ends of the first coil are configured to pass through the common threading hole to facilitate the manufacturing of the loop.

Preferably, the threading hole through which the first end of the first coil passes is configured to pass is different from the threading hole through which the second end of the first coil is configured to pass, and the threading hole through which the first end of the first coil is configured to pass is the same as or different from the threading hole through which the third end of the second coil is configured to pass, and the threading hole through which the second end of the first coil is configured to pass is the same as or different from the threading hole through which the third end of the second coil is configured to pass. The beneficial effects include: the threading hole through which the first end of the first coil is configured to pass is different from the threading hole through which the second end of the first coil is configured to pass, so that the contact area between the first end of the first coil and the fixing plate and between the second end of the first coil and the fixing plate is increased, the tension between the first end of the first coil and the second end of the first coil is increased, and thus the friction force between the first end of the first coil and the fixing plate and between the second end of the first coil and the fixing plate is increased.

Preferably, the threading hole through which the third end of the second coil is configured to pass is different from the threading hole through which the fourth end of the second coil is configured to pass, and the threading hole through which the third end of the second coil is configured to pass and the threading hole through which the fourth end of the second coil is configured to pass are arranged adjacently or arranged at intervals. The beneficial effects include: the providing of the number of the threading holes can be reduced by arranging the threading hole through which the third end of the second coil is configured to pass and the threading hole through which the fourth end of the second coil is configured to pass are arranged adjacently, thus reducing the size of the fixing plate; and the friction force between the fourth end of the second coil and the fixing plate can be increased by arranging the threading hole through which the third end of the second coil passes and the threading hole through which the fourth end of the second coil passes at intervals, thus increasing the anti-retreating effect.

Preferably, the fourth end of the second coil, after being compressed by the fixing portion, is further movably connected to the second end of the first coil or the fixing portion to form a third adjustable portion. The beneficial effects include: the second coil is further prevented from being retreated under the pulling force of the graft.

Preferably, the suture is any one of a silk suture, a catgut suture, a chemical synthesis suture, and a pure natural collagen suture. The beneficial effects include: the suture is selected to meet the stress strength according to the clinic need for stress.

Preferably, the fixing plate is further provided with at least one traction hole. The beneficial effects include: the fixing plate is provided with the traction hole to facilitate the providing of a traction rope in clinic to pull the loop to an accurate position.

Preferably, the fixing plate is further provided with at least one loop overturning hole. The beneficial effects include: the fixing plate is provided with the loop overturning hole to facilitate a loop overturning line in clinic is used to overturn and adjust the fixing plate to an accurate angle and position.

Preferably, the fixing plate is any one of a titanium plate or a PEEK.

In a second aspect, a manufacturing method of a loop is further provided by the present invention, comprising:
S1, providing a fixing plate and a suture, wherein the fixing plate is provided with at least two threading holes;
S2, enabling the suture to pass through the threading hole to form a first coil on a second side of the fixing plate, movably connecting a first end of the first coil to a second end of the first coil to form a first adjustable portion on a first side of the fixing plate;
S3, enabling the first end of the first coil to be wound to form a fixing portion on the first side of the fixing plate and to pass through a first threading hole to form a second coil on the second side of the fixing plate; and
S4, enabling the fourth end of the second coil to pass through a second threading hole and to be compressed by the fixing portion and the fixing plate to form a second adjustable portion.

The manufacturing method of the loop has the following beneficial effects: the loop is woven by one suture, and the first coil and the second coil are used to fix a graft to guarantee the overall stress strength of the loop; the length of the coil in the loop can be automatically adjustable by the first adjustable portion under the action of the graft to guarantee that the loop length of the first coil is the same as the coil length of the second coil, so that no redundant calculation is required during surgery, the surgery duration is shortened, and the surgical risk is reduced; moreover, the coil length of the second coil can be movably adjustable by the second adjustable portion under the action of artificial external force, and then the coil length of the first coil is movably adjustable by the first adjustable portion. As compared with a structure with two ends capable of being adjusted and contracted, the loop of the present invention is more stable, and less prone to inconsistent contraction on the two sides; the second adjustable portion can achieve self-locking under the action of the pulling force of the graft, which not only can effectively prevent the second coil from being retreated, but also can achieve self-locking without the operation of external force, and the duration of surgery is saved. On the other hand, it is guaranteed that the first coil cannot be enlarged and retreated by the action of pulling force of the graft without the action of artificial external force, so that the entire loop cannot retreat under the reaction force of the graft, the smooth surgery is guaranteed and the surgical risk is reduced.

Preferably, the step S2 comprises: enabling the suture to be wound to form the first coil and movably connecting the first end of the first coil to the second end of the first coil to form the first adjustable portion, and then enabling the first coil from the first side of the fixing plate to pass through the second threading hole or a third threading hole to the second side of the fixing plate. The beneficial effects include: the weaving step is simpler to facilitate the manufacturing of the loop.

Preferably, the step S2 comprises:
S21, enabling one end of the suture from the first side of the fixing plate to pass through any one of the first threading hole, the second threading hole and the third threading hole to the second side of the fixing plate to form the first coil; and
S22, enabling the first end of the first coil to pass through a threading hole, different from the threading hole in the step S21, to the first side of the fixing plate, and then movably connecting the first end of the first coil to the second end of the first coil at the first side of the fixing plate to form the first adjustable portion.

The beneficial effects include: the threading hole through which the first end of the first coil is configured to pass is different from the threading hole through which the second end of the first coil is configured to pass, so that the contact area between the first end of the first coil and the fixing plate and between the second end of the first coil and the fixing plate is increased, the tension between the first end of the first coil and the second end of the first coil is increased, and thus the friction force between the first end of the first coil and the fixing plate and between the second end of the first coil and the fixing plate is increased.

### BRIEF DESCRIPTION OF THE DRAWINGS

FIG. 1 is a structure diagram of a fixed-length type titanium plate with a loop in the prior art.
FIG. 2 is a structure diagram of an adjustable type titanium plate with a loop in the prior art;
FIG. 3 is a structure diagram of a first loop of the present invention;
FIG. 4 is a structure diagram of a second loop of the present invention;
FIG. 5 is a diagram of a use state of the first loop of the present invention;
FIG. 6 is a diagram of one connection of a first end of a first coil and a second end of the first coil of the present invention;
FIG. 7 is a diagram of another connection of a first end of a first coil and a second end of the first coil of the present invention;
FIG. 8 is a diagram of a fixing plate of the present invention;
FIG. 9 is a structure diagram formed in a manufacturing process of the first loop of the present invention;
FIG. 10 is a structure diagram formed in a manufacturing process of the second loop of the present invention.

### DESCRIPTION OF THE EMBODIMENTS

In order to make objectives, technical solutions, and advantages of the present invention clearer, the technical solutions in the present invention are described clearly and completely in the following with reference to accompanying drawings in the embodiments of the present invention. Apparently, the described embodiments are only part rather than all of the embodiments of the present invention. Based on the embodiments of the present invention, all the other embodiments obtained by those of ordinary skill in the art without inventive effort are within the scope of the present invention. Unless otherwise mentioned, all technical and scientific terms used herein have the same meaning as commonly understood by one of ordinary skill in the art to which the present invention belong. "Comprise", "include" and the like are intended to denote the element or object before the word is included in the listed element or object and thereof equivalents after the word, instead of excluding other elements or objects.

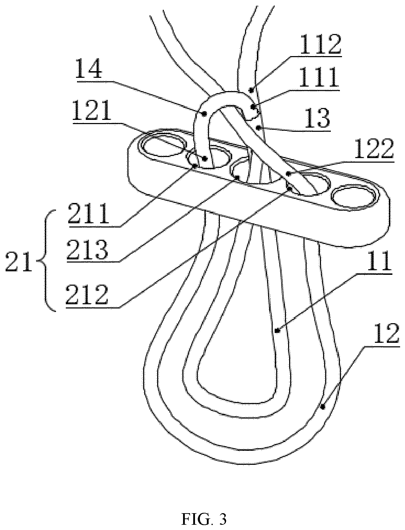

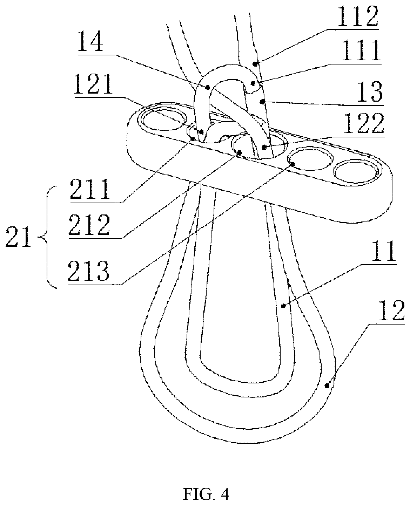

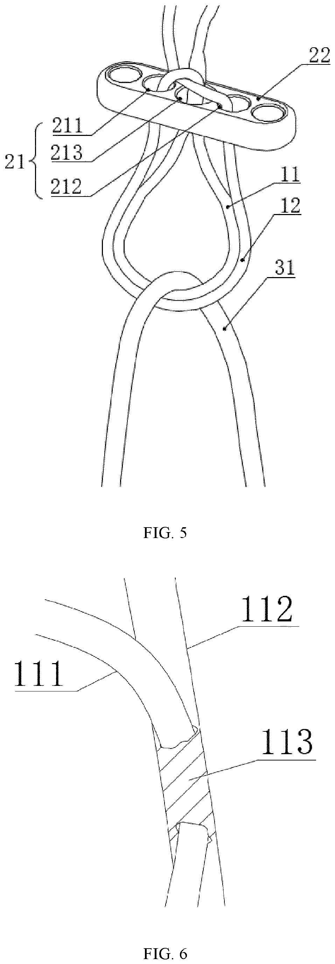

FIG. 3 is a structure diagram of a first loop of the present invention; FIG. 4 is a structure diagram of a second loop of the present invention; and FIG. 5 is a diagram of a use state of the first loop of the present invention.

To solve the problems in the prior art, referring to FIG. 3, FIG. 4 and FIG. 5, a loop is provided in an embodiment of the present invention, comprising a fixing plate (not labeled in figure) and a suture (not labeled in figure), wherein the fixing plate (not labeled in figure) is provided with at least two threading holes 21, the suture (not labeled in figure) is wound to form a first coil 11 and a second coil 12, the first coil 11 and the second coil 12 are arranged on the same side of the fixing plate (not labeled in figure) to fix a graft 31, so that the overall stress strength of the loop is increased, and the loop becomes firmer. Due to the graft 31 simultaneously passing through the first coil 11 and the second coil 12, the clinical requirement on structural mechanics can be met, and the loop can be suitable for fixing the grafts with different tensile forces. Two ends of each of the first coil 11 and the second coil 12 are configured to pass through the threading holes 21, and the two ends of the first coil 11 are respectively defined as a first end 111 and a second end 112, the first end 111 of the first coil 11 and the second end 112 of the first coil 11 are movably connected therewith to form a first adjustable portion 13, the two ends of the second coil 12 are respectively defined as a third end 121 and a fourth end 122, the third end 121 of the second coil 12 is continuously connected to the first end 111 of the first coil 11 to form a fixing portion 14, wherein the fixing portion 14 is used to compress the fourth end 122 of the second coil 12 in order to form a second adjustable portion (not labeled in figure). The graft 31 is to be fixed on the first coil 11 and the second coil 12 so as to apply acting force on the first coil 11 and the second coil 12. The graft 31 applies the acting force on the second coil 12, on the one hand, results in that the fixing portion 14 is to be contracted under the traction of the third end 121 of the second coil 12 so as to compress the fourth end 122 of the second coil 12 against the fixing plate (not labeled in the figure) in order to lock the second coil 12 with a determined length thereof. Moreover, the fourth end 122 of the second coil 12 is compressed between the fixing portion 14 and the fixing plate (not labeled in figure) to make the suture of the fourth end 122 of the second coil 12 generate friction with the fixing portion 14 and the fixing plate (not labeled in figure), thus further preventing the second coil 12 from being retreated, i.e., the second adjustable portion (not labeled in figure) can form dual locking on the fourth end 122 of the second coil 12 so as to prevent the second coil 12 from being retreated. Thus, the second adjustable portion (not labeled in figure) can achieve self-locking under the action of pulling force of the graft 31, which not only can effectively prevent the second coil 12 from being retreated, but also can achieve self-locking without the operation of external force, and the duration of surgery is saved. On the other hand, as the first coil 11 and the second coil 12,which are continuously connected to two respective ends of the first end 111 of the first coil 11, are to fix the same graft 31, it is guaranteed that the first coil 11 cannot be enlarged and retreated under the action of the pulling force of the graft 31 when no artificial external force is applied, the whole loop cannot retreat under the reaction force of the graft 31, and thus the surgery operation is guaranteed to be gone smoothly. Due to the first adjustable portion 13 formed by movably connecting the first end 111 of the first coil 11 to the second end 112 of the first coil 11, the first end 111 of the first coil 11 can be movable with respective to the second end 112 of the first coil 11, the coil length of the first coil 11 and the coil length of the second coil 12 can be automatically adjusted to be the same through the first adjustable portion 13 under the action of the graft 31 during surgery. During surgery, as the coil length of the first coil 11 and the coil length of the second coil 12 need to be adjusted to a lesser extent, and the length of the coil in the whole loop can be automatically and slightly adjustable by the first adjustable portion 13 under the action of the graft 31 to guarantee that the coil length of the first coil 11 can be the same as the coil length of the second coil 12, so that no redundant calculation is required during surgery, the duration of surgery is shortened, and the surgical risk is reduced. Moreover, as the first coil 11 and the second coil 12 are set to have the same coil length, the pulling force of the graft 31 can be shared by the first coil and the second coil 12 when the graft 31 is in use, the stress strength of the loop is increased, the service life of the loop is prolonged, and a situation is avoided that the coil is easy to break if the first coil 11 and the second coil 12 are designed to have different coil lengths and only one coil is stressed, which causes the coil to break easily. And before surgery, the coil length of the second coil 12 can be movably adjustable by the second adjustable portion (not labeled in figure) under the action of artificial external force, and then the coil length of the first coil 11 is movably adjustable by the first adjustable portion 13. And meanwhile, due to the providing of the first adjustable portion 13, the situation is also avoided that the coil lengths of the first coil 11 and the second coil 12 can be automatically changed without external force when the coil length needs to be adjusted for use during surgery, and thus the time of preparation before surgery can be saved.

In an embodiment of the present invention, the two ends of the first coil 11 are respectively defined as the first end 111 and the second end 112, the first coil 11 is a suture segment located at the second side (not labeled in figure) of the fixing plate (not labeled in figure), the first end 111 of the first coil 11 and the second end 112 of the first coil 11 are respectively suture segments, which are to penetrate through the threading holes 21 by the two ends of the first coil 11 and located at the first side 22 of the fixing plate (not labeled in figure). The two ends of the second coil 12 are respectively defined as the third end 121 and the fourth end 122, the second coil 12 is a suture segment which is located at the second side (not labeled in figure) of the fixing plate (not labeled in figure), and the third end 121 of the second coil 12 and the fourth end 122 of the second coil 12 are respectively suture segments, which are to penetrate through the threading holes 21 by the two ends of the second coil 12 and located at the first side 22 of the fixing plate (not labeled in figure).

In some embodiments of the present invention, referring to FIG. 3, FIG. 4 and FIG. 5, the first coil 11 and the second coil 12 are woven by one continuous suture (not labeled in figure) to pass through the threading holes 21 of the fixing plate (not labeled in figure), so that the first coil 11, the second coil 12 and the fixing portion 14 are of a continuous suture, and the loop becomes firmer and more durable, thus preventing the joint of the coil from being broken to affect the use.

In some embodiments of the present invention, the two ends of the first coil 11 are configured to pass through the same or common threading hole 21, and the threading hole 21 or holes through which the two ends of the first coil 11 are to pass is different from the threading hole 21 through which the third end 121 of the second coil 12 is to pass, the threading hole 21 through which the third end 121 of the second coil 12 is to pass is different from the threading hole 21 through which the fourth end 122 of the second coil 12 is to pass, and the threading hole 21 through which the third end 121 of the second coil 12 is to pass and the threading hole 21 through which the fourth end 122 of the second coil 12 is to pass can be arranged adjacently or arranged at intervals.

In some embodiments of the present invention, referring to FIG. 3 and FIG. 5, the two ends of the first coil 11 are to simultaneously pass through the third threading hole 213, the third end 121 of the second coil 12 is to pass through the first threading hole 211, the fourth end 122 of the second coil 12 is to pass through the second threading hole 212, and the first threading hole 211, the third threading hole 213 and the second threading hole 212 are arranged in sequence, and the second threading hole 212 and the first threading hole 211 are arranged at intervals to increase the friction force between the fourth end 122 of the second coil 12 and the fixing plate (not labeled in figure), thus increasing the anti-retreating effect.

In some other embodiments of the present invention, the two ends of the first coil 11 are to simultaneously pass through the third threading hole 213, the third end 121 of the second coil 12 is to pass through the first threading hole 211, the fourth end 122 of the second coil 12 is to pass through the second threading hole 212, the first threading hole 211, the third threading hole 213 and the second threading hole 212 are arranged in sequence, and at least one threading hole 21 is further provided between the third threading hole 213 and the second threading hole 212 to further increase the friction force between the fourth end 122 of the second coil 12 and the fixing plate, thus increasing the anti-retreating effect.

In some further embodiments of the present invention, the two ends of the first coil 11 are to simultaneously pass through the third threading hole 213, the third end 121 of the second coil 12 is to pass through the first threading hole 211, the fourth end 122 of the second coil 12 is to pass through the second threading hole 212, and the first threading hole 211 and the second threading hole 212 are arranged adjacently, for example, the third threading hole 213, the first threading hole 211 and the second threading hole 212 are arranged in sequence, or the third threading hole 213, the second threading hole 212 and the first threading hole 211 are arranged in sequence.

In some other embodiments of the present invention, the two ends of the first coil 11 are to simultaneously pass through the third threading hole 213, the third end 121 of the second coil 12 is to pass through the first threading hole 211, the fourth end 122 of the second coil 12 is to pass through the third threading hole 213, and the third threading hole 213 and the first threading hole 211 are arranged adjacently, so that the providing of the number of the threading holes can be reduced, and the size of the fixing plate is reduced; or the third threading hole 213 and the first threading hole 211 are arranged at intervals, i.e., at least one threading hole is further provided between the third threading hole 213 and the first threading hole 211 to increase the friction force between the fourth end 122 of the second coil 12 and the fixing plate, thus increasing the anti-retreating effect.

In some embodiments of the present invention, the threading hole 21 through which the first end 111 of the first coil 11 is to pass is different from the threading hole 21 through which the second end 112 of the first coil 11 is to pass, and the threading hole 21 through which the first end 111 of the first coil 11 is to pass may be the same as or different from the threading hole 21 through which the third end 121 of the second coil 12 is to pass, the threading hole 21 through which the second end 112 of the first coil 11 is to pass may be the same as or different from the threading hole 21 through which the third end 121 of the second coil 12 is to pass, the threading hole 21 through which the third end 121 of the second coil 12 is to pass is different from the threading hole 21 through which the fourth end 122 of the second coil 12 is to pass, and the threading hole 21 through which the third end 121 of the second coil 12 is to pass and the threading hole 21 through which the fourth end 122 of the second coil 12 is to pass may be arranged adjacently or at intervals. The threading hole 21 through which the first end 111 of the first coil 11 is to pass is different from the threading hole 21 through which the second end 112 of the first coil 11 is to pass, so that the contact area between the first end 111 of the first coil 11 and the fixing plate (not labeled in figure) and between the second end 112 of the first coil 11 and the fixing plate is increased, the tension between the first end part 111 of the first coil 11 and the second end 112 of the first coil 11 is increased, and the friction force between the first end 111 of the first coil 11 and the fixing plate and between the second end 112 of the first coil 11 and the fixing plate is increased.

In some embodiments of the present invention, referring to FIG. 4, the first end 111 of the first coil 11 is configured to pass through the first threading hole 211, the second end 112 of the first coil 11 is to pass through the second threading hole 212, the third end 121 of the second coil 12 is to pass through the first threading hole 211, the fourth end 122 of the second coil 12 is to pass through the second threading hole 212, and the second threading hole 212 and the first threading holes 211 are arranged adjacently, so that the providing of the number of the threading holes can be reduced, and the size of the fixing plate is reduced.

In some embodiments of the present invention, the first end 111 of the first coil 11 is to pass through the first threading hole 211, the second end 112 of the first coil 11 is to pass through the second threading hole 212, the third end 121 of the second coil 12 is to pass through the first threading hole 211, the fourth end 122 of the second coil 12 is to pass through the second threading hole 212, and the second threading hole 212 and the first threading hole 211 are arranged at intervals, i.e., at least one threading hole 21 is further provided between the second threading hole 212 and the first threading hole 211 to increase the friction force between the fourth end 122 of the second coil 12 and the fixing plate, thus increasing the anti-retreating effect and increasing the friction force between the two ends of the first coil 11 and the fixing plate.

In some embodiments of the present invention, the first end 111 of the first coil 11 is to pass through the first threading hole 211, the second end 112 of the first coil 11 is to pass through the second threading hole 212, the third end 121 of the second coil 12 is to pass through the first threading hole 211, the fourth end 122 of the second coil 12 is to pass through a third threading hole 213 to increase the friction between the fourth end 122 of the second coil 12 and the fixing plate, thus increasing the anti-retreating effect. The arrangement sequence of the first threading hole 211, the second threading hole 212 and the third threading hole 213 can be not limited, and it can be that the first threading hole 211, the second threading hole 212 and the third threading hole 213 are arranged in sequence, or the first threading hole 211, the third threading hole 213 and the second threading hole 212 are arranged in sequence, or the second threading hole 212, the first threading hole 211 and the third threading hole 213 are arranged in sequence, and the like, unnecessary details are not given herein.

In some embodiments of the present invention, the first end 111 of the first coil 11 is to pass through the first threading hole 211, the second end 112 of the first coil 11 is to pass through the second threading hole 212, the third end 121 of the second coil 12 is to pass through the first threading hole 211, the fourth end 122 of the second coil 12 is to pass through the third threading hole 213, the first threading hole 211, the second threading hole 212 and the third threading hole 213 are arranged in sequence, and at least one threading hole can be further provided between the third threading hole 213 and the second threading hole 212 to further increase the friction force between the fourth end 122 of the second coil 12 and the fixing plate, thus increasing the anti-retreating effect.

In some embodiments of the present invention, the first end 111 of the first coil 11 is to pass through the first threading hole 211, the second end 112 of the first coil 11 is to pass through the second threading hole 212, the third end 121 of the second coil 12 is to pass through the third threading hole 213, the fourth end 122 of the second coil 12 is to pass through the first threading hole 211, the second threading hole 212 or a fourth threading hole. The arrangement sequence of the first threading hole 211, the second threading hole 212, the third threading hole 213 and the fourth threading hole can be not limited, it can be that the first threading hole 211, the second threading hole 212, the third threading hole 213 and the fourth threading hole are arranged in sequence, or the third threading hole 213, the first threading hole 211, the second threading hole 212 and the fourth threading hole are arranged in sequence, and the like, unnecessary details are not given herein.

FIG. 6 is a diagram of a first connection of a first end of a first coil and a second end of the first coil of the present invention. In some embodiments of the present invention, referring to FIG. 6, the second end 112 of the first coil 11 is provided with a nesting portion 113, the first end 111 of the first coil 11 is configured to pass through the nesting portion 113 to be movably connected to the second end 112 of the first coil 11 so as to form the first adjustable portion (not labeled in figure), the nesting portion 113 is made of the same material as the suture, and the nesting portion 113 is in interference fit with the first end 111 of the first coil 11, the anti-retreating effect can be obtained through the friction force between the nesting portion 113 and the first end 111 of the first coil 11, and the first end 111 of the first coil 11 can pass through the nesting portion 113 simply and easily, thus facilitating the manufacturing of the loop.

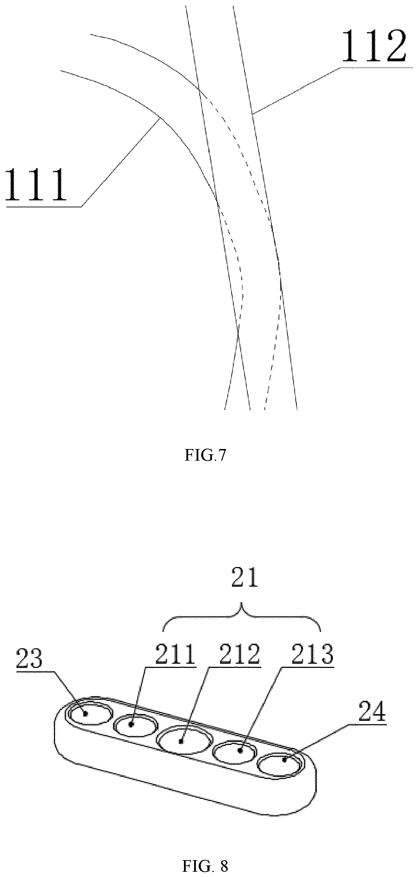

FIG. 7 is a diagram of a second connection of the first end of the first coil and the second end of the first coil of the present invention. In some better embodiments of the present invention, referring to FIG. 7, the first end 111 of the first coil 11 is movably connected to the second end 112 of the first coil 11 through a threading process to form the first adjustable portion (not labeled in figure), i.e., a suture of the first end 111 of the first coil 11 is configured to entirely pass through the internal of a suture segment of the second end 112 of the first coil 11 through the threading process, so that the first end 111 of the first coil 11 is movably connected to the second end 112 of the first coil 11 to form the first adjustable portion (not labeled in figure). Due to the weaving or knitting process of the suture itself, the outer surface roughness of the suture is high, a plurality of line holes actually exist in an interior of the suture, and the line holes in the suture are also of rough inner surfaces. By means of the threading process, the outer surface of the suture of the first end 111 of the first coil 11 is in interference fit with the line holes in the suture of the second end 112 of the first coil 11, the anti-retreating effect is achieved by mutual friction force, the threading process is simple, and there is no need to additionally provide a fixing structure for fixing the first end 111 of the first coil 11 to the second end 112 of the first coil 11, so that the structure is simpler, and the overall size of the loop is reduced.

In some other embodiments of the present invention, the first end of the first coil is movably connected to the second end of the first coil by a binding method to form the first adjustable portion. Due to the first end of the first coil and the second end of the first coil are movably connected therewith by a binding method, so that the first adjustable portion at the joint of the first end of the first coil and the second end of the first coil has a certain friction force thereof with no lose of the moving capacity, and the coil length of the first coil and the coil length of the second coil can be automatically adjusted to be the same by the first adjustable portion under the action of the graft during surgery. The specific binding method is the conventional means in this field, and unnecessary details are not given herein.

In some other embodiments of the present invention, the first end of the first coil and the second end of the second coil are both provided with fasteners, the first end of the first coil is movably connected to the second end of the first coil by a fastening method to form the first adjustable portion, and a specific structure of the fastener is the conventional means of this field, and unnecessary details are not given herein. The fastener can make the first end of the first coil have no lose of the moving capacity with respect to the second end of the first coil and forms the locking force on the first end of the first coil and the second end of the first coil, so that the coil length of the first coil and the coil length of the second coil can be automatically adjusted to be the same by the first adjustable portion under the action of the graft during surgery.

In some further embodiments, the first end of the first coil and the second end of the first coil are both provided with clamps, the first end of the first coil is movably connected to the second end of the first coil by a clamping method to form the first adjustable portion, and a specific structure of the clamp is the conventional means of this field, and unnecessary details are not given herein. The clamp can make the first end of the first coil have no lose of the moving capacity with respect to the second end of the first coil and forms the locking force on the first end of the first coil and the second end of the first coil, so that the coil length of the first coil and the coil length of the second coil can be automatically adjusted to be the same by the first adjustable portion under the action of the graft during surgery.

In some embodiments of the present invention, the fourth end 122 of the second coil 12, after being compressed by the fixing portion 14, is further movably connected to the second end 112 of the first coil 11 or the fixing portion 14 to form a third adjustable portion, and the providing of the third adjustable portion can further prevent the second coil 12 from being retreated under the pulling force of the graft 31. The forming process of the third adjustable portion is the same as the forming process of the first adjustable portion, i.e., the second end of the first coil or the fixing portion is provided with a nesting portion, the fourth end of the second coil, after being compressed by the fixing portion, is to pass through the nesting portion to be movably connected to the second end of the first coil or the fixing portion to form the third adjustable portion; or the fourth end of the second coil, after being compressed by the fixing portion, is movably connected to the second end of the first coil or the fixing portion through a threading process to form the third adjustable portion; or the fourth end of the second coil, after being compressed by the fixing portion, is movably connected to the second end of the first coil or the fixing portion through any one of fastening, clamping or binding method to form the third adjustable portion.

In some embodiments of the present invention, the suture is any one of a silk suture, a catgut suture, a chemical synthesis suture, and a pure natural collagen suture, and the suture conforming to the stress strength is selected according to the clinic need for stress.

In some embodiments of the present invention, the suture is any one of a polymer polyethylene surgical suture, a nylon suture, a polyester suture, a polyamide suture, and a polypropylene suture.

FIG. 8 is a diagram of a fixing plate. In some better embodiments of the present invention, referring to FIG. 8, the fixing plate (not labeled in figure) is further provided with at least one traction hole 23, the traction hole 23 is provided to facilitate a traction rope in clinic to pull the loop to an accurate position.

In some better embodiments of the present invention, referring to FIG. 8, the fixing plate (not labeled in figure) is further provided at least one loop overturning hole 24, and the loop overturning hole 24 is provided to facilitate a loop overturning line to overturn and adjust the fixing plate to an accurate angle and position.

In some embodiments of the present invention, the threading holes of the fixing plate have apertures with same diameters.

In some embodiments of the present invention, referring to FIG. 8, part or all of threading holes 21 of the fixing plate (not labeled in figure) have apertures with different diameters, the threading holes 21 comprise a first threading hole 211, a second threading hole 212, and a third threading hole 213, and a diameter of the aperture of the second threading hole 212 is greater than that of the aperture of the first threading hole 211 and that of the aperture of the third threading hole 213, thus facilitating a plurality of sutures or suture segments to pass through the second threading hole 212.

In some embodiments of the present invention, the fixing plate is any one of a titanium plate and a PEEK, and PEEK is polyether-ether-ketone.

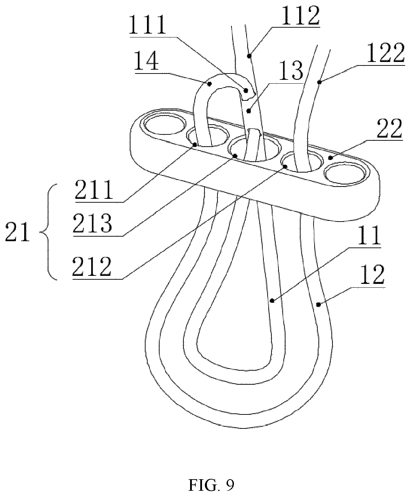

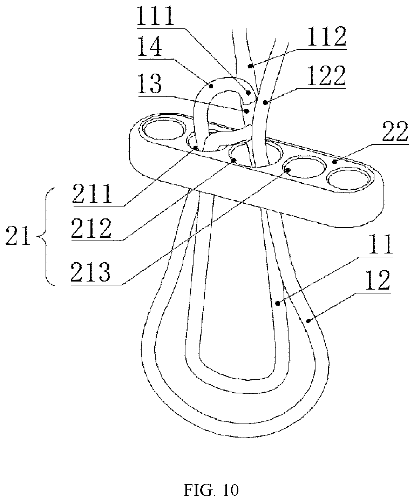

FIG. 9 is a structure diagram formed in a manufacturing process of a first loop of the present invention, and FIG. 10 is a structure diagram formed in a manufacturing process of a second loop of the present invention.

In some embodiments of the present invention, referring to FIG. 3, FIG. 4, FIG. 9, and FIG. 10, a manufacturing method of a loop is provided, comprising:
S1, providing a fixing plate (not labeled in figure) and a suture (not labeled in figure), wherein the fixing plate (not labeled in figure) is provided with at least two threading holes 21;
S2, enabling the suture (not labeled in figure) to pass through the threading hole 21 to form a first coil 11 on a second side (not labeled in figure) of the fixing plate, and movably connecting a first end 111 of the first coil 11 to a second end 112 of the first coil 11 to form a first adjustable portion 13 on a first side 22 of the fixing plate (not labeled in figure);
S3, enabling the first end of the first coil to be wound to form a fixing portion 14 on the first side 22 of the fixing plate and then to pass through a first threading hole 211 to form a second coil 12 on a second side (not labeled in figure) of the fixing plate; and
S4, enabling a fourth end 122 of the second coil 12 to pass through a second threading hole 212 and then to be compressed by the fixing portion 14 and the fixing plate (not labeled in figure) to form a second adjustable portion (not labeled in figure).

In some embodiments of the present invention, referring to FIG. 9, the step S2 comprises: enabling the suture (not labeled in figure) to be wound to form the first coil 11 and movably connecting the first end 111 of the first coil 11 to the second end 112 of the first coil 11 to form a first adjustable portion 13, and then enabling the first coil 11 from the first side 22 of the fixing plate (not labeled in figure) to pass through a third threading hole 213 to the second side (not labeled in figure) of the fixing plate, wherein the first threading hole 211, the third threading hole 213 and the second threading hole 212 are arranged in sequence. Further, the first threading hole 211, the third threading hole 213 and the second threading hole 212 can also be arranged in other modes, unnecessary details are not given herein.

In some embodiments of the present invention, the step S2 comprises: enabling the suture (not labeled in figure) to be wound to form a first coil 11 and movably connecting a first end 111 of the first coil 11 to a second end 112 of the first coil 11 to form a first adjustable portion 13, and then enabling the first coil 11 from the first side 22 of the fixing plate (not labeled in figure) to pass through a second threading hole 212 to the second side (not labeled in figure) of the fixing plate, wherein the first threading hole 211 and the second threading hole 212 can be arranged adjacently or arranged at intervals, unnecessary details are not given herein.

In some embodiments of the present invention, the step S2 comprises:
S21, enabling one end of the suture (not labeled in figure) from the first side 22 of the fixing plate (not labeled in figure) to pass through any one of the first threading hole 211, the second threading hole 212 and the third threading hole 213 to the second side (not labeled in figure) of the fixing plate (not labeled in figure) to form the first coil 11; and
S22, enabling the first end 111 of the first coil 11 to pass through a threading hole 21, different from the threading hole in the step S21, to the first side 22 of the fixing plate (not labeled in figure), and movably connecting the first end of the first coil to the second end 112 of the first coil 11 at the first side 22 of the fixing plate (not labeled in figure) to form a first adjustable portion 13.

In some embodiments, referring to FIG. 10, the step S2 comprises:
S21, enabling one end of the suture (not labeled in figure) from the first side 22 of the fixing plate (not labeled in figure) to pass through the second threading hole 212 to the second side (not labeled in figure) of the fixing plate (not labeled in figure) to form the first coil 11; and
S22, enabling the first end 111 of the first coil 11 to pass through the first threading hole 211 to the first side 22 of the fixing plate (not labeled in figure), and movably connecting the first end of the first coil to the second end 112 of the first coil 11 that is located at the first side 22 of the fixing plate (not labeled in figure) to form a first adjustable portion 13. Further, the first threading hole 211 and the second threading hole 212 can be arranged adjacently or arranged at intervals, unnecessary details are not given herein.

While the embodiments of the present invention have been described in detail, it will be apparent to those skilled in the art that various modifications and variations can be made to the embodiments. However, it is to be understood that such modifications and variations are within the scope and spirit of the present invention as described in the appended claims. Furthermore, the present invention described herein is susceptible to other embodiments and may be embodied or carried out in various ways.

## Claims

1. A loop, comprising a fixing plate and a suture, wherein the fixing plate is provided with at least two threading holes, the suture is wound to form a first coil and a second coil, the first coil and the second coil are arranged on a same side of the fixing plate, and two ends of each of the first coil and the second coil are configured to pass through the threading holes; the two ends of the first coil are respectively defined as a first end and a second end, and the first end of the first coil and the second end of the first coil are movably connected therewith to form a first adjustable portion; the two ends of the second coil are respectively defined as a third end and a fourth end, the third end of the second coil is connected to the first end of the first coil to form a fixing portion, and the fixing portion is configured to compress the fourth end of the second coil to form a second adjustable portion.

2. The loop according to claim 1, wherein the first coil and the second coil are woven by a continuous suture configured to pass through the threading holes of the fixing plate.

3. The loop according to claim 1, wherein the second end of the first coil is provided with a nesting portion, and the first end of the first coil is movably connected to the second end of the first coil by passing through the nesting portion to form the first adjustable portion.

4. The loop according to claim 1, wherein the first end of the first coil and the second end of the first coil are movably connected by a threading process to form the first adjustable portion.

5. The loop according to claim 1, wherein the first end of the first coil and the second end of the first coil are movably connected by a fastening, clamping or binding method to form the first adjustable portion.

6. The loop according to claim 1, wherein the two ends of the first coil are configured to pass through a common threading hole, and the threading hole through which the two ends of the first coil are configured to pass is different from the threading hole through which the third end of the second coil is configured to pass.

7. The loop according to claim 1, wherein the threading hole through which the first end of the first coil is configured to pass is different from the threading hole through which the second end of the first coil is configured to pass, and the threading hole through which the first end of the first coil is configured to pass is the same as or different from the threading hole through which the third end of the second coil is configured to pass, and the threading hole through which the second end of the first coil is configured to pass is the same as or different from the threading hole through which the third end of the second coil is configured to pass.

8. The loop according to claim 6 or 7, wherein the threading hole through which the third end of the second coil is configured to pass is different from the threading hole through which the fourth end of the second coil is configured to pass, and the threading hole through which the third end of the second coil is configured to pass and the threading hole through which the fourth end of the second coil is configured to pass are arranged adjacently or arranged at intervals.

9. The loop according to claim 1, wherein the fourth end of the second coil, after being compressed by the fixing portion, is further movably connected to the second end of the first coil or the fixing portion to form a third adjustable portion.

10. The loop according to claim 1, wherein the suture is any one of a silk suture, a catgut suture, a chemical synthesis suture, and a pure natural collagen suture.

11. The loop according to claim 1, wherein the fixing plate is further provided with at least one traction hole.

12. The loop according to claim 1, wherein the fixing plate is further provided with at least one loop overturning hole.

13. The loop according to claim 1, wherein the fixing plate is any one of a titanium plate and a PEEK plate.

14. A manufacturing method of a loop, comprising:
S1, providing a fixing plate and a suture, wherein the fixing plate is provided with at least two threading holes;
S2, enabling the suture to pass through the threading hole to form a first coil on a second side of the fixing plate, movably connecting a first end of the first coil to a second end of the first coil to form a first adjustable portion on a first side of the fixing plate;
S3, enabling the first end of the first coil to be wound to form a fixing portion on the first side of the fixing plate and to pass through a first threading hole to form a second coil on the second side of the fixing plate; and S4, enabling the fourth end of the second coil to pass through a second threading hole and to be compressed by the fixing portion and the fixing plate to form a second adjustable portion.

15. The manufacturing method of the loop according to claim 14, wherein the step S2 comprises: enabling the suture to be wound to form the first coil and movably connecting the first end of the first coil to second end of the first coil to form the first adjustable portion, and then enabling the first coil from the first side of the fixing plate to pass through the second threading hole or a third threading hole to the second side of the fixing plate.

16. The manufacturing method of the loop according to claim 14, wherein the step S2 comprises:
S21, enabling one end of the suture from the first side of the fixing plate to pass through any one of the first threading hole, the second threading hole and the third threading hold to the second side of the fixing plate to form the first coil; and
S22, enabling the first end of the first coil to pass through a threading hole, different from the threading hole in the step S21, to the first side of the fixing plate, and then movably connecting the first end of the first coil to the second end of the first coil at the first side of the fixing plate to form the first adjustable portion.
